# EUROPEAN PATENT APPLICATION

(11) **EP 4 393 485 A1**
(43) Date of publication of application: **03.07.2024**
(21) Application number: 22868844.6
(22) Date of filing: 26.07.2022
(51) Int. Cl.: A61K 9/72, A61K 31/702, A61P 11/08, A61M 11/00

(54) **PHARMACEUTICAL ASSEMBLY COMPRISING TOBRAMYCIN INHALATION SOLUTION AND USE THEREOF**

(30) Priority: 18.09.2021 CN 202111095755
(71) Applicant: Joincare Pharmaceutical Group Industry Co., Ltd., Shenzhen City, Guangdong 518057 (CN); Guangzhou Joincare Respiratory Drug Engineering Technology Co., Ltd., Guangzhou City, Guangdong 510530 (CN)
(72) Inventor: JIN, Fang, Shenzhen City, Guangdong 518057 (CN); HUANG, Juan, Guangzhou City, Guangdong 510530 (CN); DENG, Wei, Guangzhou City, Guangdong 510530 (CN); YANG, Xia, Shanghai 201203 (CN); LI, Min, Shanghai 201203 (CN); YU, Xiong, Shenzhen City, Guangdong 518057 (CN)
(74) Representative: Schiweck Weinzierl Koch Patentanwälte Partnerschaft mbB
(86) International application number: PCT/CN2022/107735
(87) International publication number: WO 2023/040467

(57) **Abstract**

A pharmaceutical assembly comprising a tobramycin inhalation solution and use thereof. The pharmaceutical assembly comprises: (a) a tobramycin inhalation solution; and (b) a vibrating screen atomizer for use in combination with a tobramycin inhalation solution, wherein a central area of a metal mesh of the vibrating screen atomizer has 1400-1800 micropores. A droplet size of the tobramycin inhalation solution is that: D₁₀ is 0.5 µm-2.5 µm, D₅₀ is 2.0 µm-4.2 µm, and D₉₀ is 6.0 µm-9.0 µm; particles having an aerodynamic particle size less than 5.39 µm have a mass percentage not less than 45% of the tobramycin inhalation solution. The pharmaceutical assembly is used for treating bronchiectasis.

## Description

### TECHNICAL FIELD

The present invention relates to the technical field of medicines, in particular to a pharmaceutical assembly comprising a tobramycin inhalation solution and use thereof.

### BACKGROUND

Aminoglycoside antibiotics are named because their molecular structures all have one amino cyclic alcohol and one or more amino sugar molecules and glycosides are formed through glycosidic bonds, are mostly polar compounds, are not easy to absorb by gastrointestinal tracts, and generally need to be injected for administration. Adverse events such as ototoxicity and nephrotoxicity usually occur clinically due to injection administration. The ototoxicity can be divided into: (1) vestibular dysfunction, which is commonly seen in streptomycin, kanamycin, gentamicin and tobramycin, the incidence rate after administration is kanamycin > streptomycin > gentamicin > tobramycin in turn; (2) cochlear nerve damage, which is commonly seen in kanamycin, amikacin, sisomicin, gentamicin and the like, the incidence rate after administration is kanamycin > amikacin > sisomicin > gentamicin > tobramycin in turn, and the incidence rate of these "subclinical deafness" reactions is about 10%-20%. The order of nephrotoxicity is kanamycin and sisomicin > gentamicin and amikacin > tobramycin and streptomycin, and generally nephrotoxicity is easy to occur after the systemic administration for more than 10 days.

Tobramycin (TOB) is an aminoglycoside antibiotic naturally produced from Streptomyces and it is bactericidal at a concentration equal to or slightly greater than its inhibitory concentration mainly by changes in the permeability of the cell membrane, progressive destruction of the cell envelope and ultimately cell death caused by inhibiting protein synthesis. It is a second generation aminoglycoside antibiotic, and compared with the first generation aminoglycoside antibiotic, it has the advantages of better curative effect, lower toxicity and less clinical drug resistance, and has become one of the most widely used aminoglycoside antibiotics in the world.

Bronchiectasis is a common chronic respiratory disease, has a long course of disease, irreversible lesions and easy to be repeatedly infected. In particular, extensive bronchiectasis can seriously damage lung tissues and functions of patients, seriously affect the life quality of the patients and simultaneously cause heavy social and economic burden.

Pseudomonas aeruginosa is a common opportunistic pathogen. Studies have shown that the pulmonary function of patients with bronchiectasis colonized by the pseudomonas aeruginosa decreases more quickly, and the disease severity is higher. Meanwhile, pseudomonas aeruginosa has the characteristics of easy colonization, easy variation and multi-drug resistance, so it is difficult to be cleared by conventional antibiotic intervention. Especially, lower respiratory tract infections such as bronchiectasis complicated with infection caused by multidrug-resistant Pseudomonas aeruginosa have high mortality and are difficult to treat.

For patients with bronchiectasis, at present, the conventional clinical treatment in China still adopts intravenous tobramycin, however, only a small amount of drugs can enter lung tissues after intravenous administration of tobramycin, and most patients still cannot eliminate pseudomonas aeruginosa after standardized oral or intravenous administration.

At present, tobramycin inhalation solution has been used to overcome the defects of oral administration or intravenous administration, but the inhalation solution is generally administered through a jet atomizer, the jet atomizer is connected with a compressor through a pipeline, the compressor forms a high-speed airflow through a fine nozzle by utilizing compressed air, the generated negative pressure drives liquid or other fluids to be sprayed onto an obstacle together, and which splash to the periphery under high-speed impact to enable liquid drops to be changed into mist-shaped particles to be ejected from an air outlet pipe. When the jet atomizer is used for administration, there are some defects, such as wide droplet size distribution, large geometric standard deviation, large volume, inconvenience in carrying, high noise, high energy consumption, large volume of residual liquid medicine, prolonged treatment time, reduced compliance.

Therefore, there is a need to develop a pharmaceutical assembly comprising a tobramycin inhalation solution to solve the above problems.

### SUMMARY

To overcome the deficiencies of the prior art, the present invention provides a pharmaceutical assembly comprising a tobramycin inhalation solution and use thereof. By using the tobramycin inhalation solution in combination with a vibrating screen atomizer, the pharmaceutical assembly comprising the tobramycin inhalation solution according to the present invention makes that the distribution of the droplet size is concentrated, the proportion of small particles reaching lungs is greatly increased, the volume of residual liquid medicine is obviously reduced, and the treatment effect on the bronchiectasis is obviously improved.

In order to achieve the purpose of the present invention, the technical solution is as follows:
In one aspect, the present invention provides a use of a pharmaceutical assembly comprising a tobramycin inhalation solution in the manufacture of a pharmaceutical product for the treatment of bronchiectasis,
wherein the pharmaceutical assembly comprises:
   (a) the tobramycin inhalation solution; and
   (b) a vibrating screen atomizer matched with the tobramycin inhalation solution;
wherein the central area of metal mesh of the vibrating screen atomizer has 1400-1800 micropores;
the droplet size of the tobramycin inhalation solution is as follows: D₁₀ is 0.5µm -2.5 µm, D₅₀ is 2.0µm -4.2 µm, and D₉₀ is 6.0µm -9.0 µm; the mass percentage of particles with an aerodynamic particle size of less than 5.39µm in the tobramycin inhalation solution is not less than 45%.

Preferably, the fine particle dose (FPD) (with an aerodynamic particle size of less than 5.39µm) of atomized particles is not less than 135mg. More preferably, the fine particle dose (FPD) (with an aerodynamic particle size of less than 5.39µm) of atomized particles is 135 mg to 270 mg.

Preferably, the central area of metal mesh of the vibrating screen atomizer has 1600-1800 micropores.

Preferably, the cross-section of the micropores in the vibrating screen atomizer is distributed in a stepped manner, and has a taper of 30-50 degrees.

Preferably, the number of the micropores having a diameter of 3.8µm -4.2 µm is not less than 80% based on the total number of the micropores.

Preferably, the vibrating screen atomizer has a vibrating frequency of 110 KHz -160 KHz.

Preferably, the vibrating screen atomizer has an atomization rate of 0.47 ml/min -0.55 ml/min.

Preferably, the vibrating screen atomizer has a current of 1A, ± 2%; and a voltage of 5V, ± 2%.

Preferably, the mass percentage of particles with an aerodynamic particle size of less than 5.39µm in the tobramycin inhalation solution is 45%∼84%.

Preferably, the droplet size of the tobramycin inhalation solution is as follows: D₁₀ is 0.5 µm-1.5 µm, D₅₀ is 3.0 µm-3.5 µm, and D₉₀ is 6.0 µm-8.5 µm.

Preferably, the tobramycin inhalation solution contains 4 % ∼ 8% (w/v) of tobramycin, 0.1%∼0.9% (w/v) of sodium chloride, and has a pH of 4.5 - 7.5.

In another aspect, the present invention provides a pharmaceutical assembly comprising a tobramycin inhalation solution, the pharmaceutical assembly comprises:
(a) the tobramycin inhalation solution; and
(b) a vibrating screen atomizer matched with the tobramycin inhalation solution;

wherein the central area of metal mesh of the vibrating screen atomizer has 1400-1800 micropores;
the droplet size of the tobramycin inhalation solution is as follows: D₁₀ is 0.5 µm-2.5 µm, D₅₀ is 2.0 µm-4.2 µm, and D₉₀ is 6.0 µm-9.0 µm; the mass percentage of particles with an aerodynamic particle size of less than 5.39µm in the tobramycin inhalation solution is not less than 45%.

Preferably, the fine particle dose (FPD) (with an aerodynamic particle size of less than 5.39µm) of atomized particles is not less than 135mg. More preferably, the fine particle dose (FPD) (with an aerodynamic particle size of less than 5.39µm) of atomized particles is 135 mg to 270 mg.

Preferably, the central area of metal mesh of the vibrating screen atomizer has 1600-1800 micropores.

Preferably, the cross-section of the micropores in the vibrating screen atomizer is distributed in a stepped manner, and has a taper of 30∼50 degrees.

Preferably, the number of the micropores having a diameter of 3.8 µm∼4.2 µm is not less than 80% based on the total number of the micropores.

Preferably, the vibrating screen atomizer has a vibrating frequency of 110 KHz∼160 KHz.

Preferably, the vibrating screen atomizer has an atomization rate of 0.47 ml/min∼0.55 ml/min.

Preferably, the vibrating screen atomizer has a current of 1A, ± 2%; and a voltage of 5V, ± 2%.

Preferably, the mass percentage of particles with an aerodynamic particle size of less than 5.39µm in the tobramycin inhalation solution is 45%∼84%.

Preferably, the droplet size of the tobramycin inhalation solution is as follows: D₁₀ is 0.5 µm-1.5 µm, D₅₀ is 3.0 µm-3.5 µm, and D₉₀ is 6.0 µm-8.5 µm.

Preferably, the tobramycin inhalation solution contains 4% ~ 8% (w/v) of tobramycin, 0.1% ∼ 0.9% (w/v) of sodium chloride, and has a pH of 4.5 - 7.5.

The vibrating screen atomizer in the present invention vibrates up and down through a vibrator, extrudes liquid medicine by using holes of a nozzle-type mesh spray head, and forms droplets by a tiny ultrasonic vibration and a mesh type spray head. The residual volume of liquid medicine is small, the atomization time is short, there is no obvious temperature rise during the atomization process, the administration volume is small, the atomization efficiency is higher, and the assembly can be carried about. Unless otherwise specified, the vibrating screen atomizer used in the present invention is conventional, and for example, as described in CN 111569200A.

When the pharmaceutical assembly is used in the clinical research of bronchiectasis, compared with the control group, the related quality of life score (QOL-B-RSS) of the test group with respiratory symptoms is obviously improved, and there was a statistically significant difference between the two groups (P < 0.001), the load value of pseudomonas aeruginosa in the test group is significantly lower than that in the control group, and there was a statistically significant difference between the two groups (P<0.001).

Compared with the prior art, the present invention has at least the following beneficial technical effects:
(1) based on the characteristics of bronchiectasis, the present invention provides a tobramycin inhalation solution and a vibrating screen atomizer matched with the tobramycin inhalation solution, the number of holes, aperture, vibration frequency, current, voltage and atomization rate in the vibrating screen atomizer are adjusted, and the droplet size, particle size parameters (D₁₀\D₅₀\D₉₀) and the like in the tobramycin inhalation solution are also controlled, this not only achieves a good clinical effect on bronchiectasis, but also has the advantages of small volume of residual liquid medicine, convenient in use, portability and low noise.
(2) The pharmaceutical assembly of the present invention not only overcomes the defects that only a small amount of drugs can enter lung tissues by oral administration or intravenous administration and cannot eliminate pseudomonas aeruginosa, but also overcomes the defects of other inhalation products such as wider droplet distribution, larger range of geometric standard deviation, inconvenient carrying by larger volume , higher noise, higher energy consumption, large volume of residual liquid medicine, prolonged treatment time, reduced compliance and the like.

### DETAILED DESCRIPTION OF THE INVENTION

In order to make the purpose, technical solutions and advantages of the present invention more apparent, the technical solutions of the present invention will be clearly and completely described below. Obviously, the described examples are only exemplary and do not represent all the technical solutions of the present application. Based on the examples of the present invention, other technical solutions obtained by those skilled in the art without making any creative effort belong to the protection scope of the present application.

In the following examples, the measurement method of the atomizer is as follows:
The data were measured by a laser particle analyzer (Sympatec GmbH) and its principle was light scattering.

An effective inhalation amount of drugs was collected by a cascade impactor having 7 stages and 1 microporous collector, and the effective inhalation amount of drugs collected was measured by a high performance liquid chromatography.

The cascade impactor connected with an L-shaped throat pipe was placed at 5 °C for 90 minutes, the effective inhalation amount of drugs was tested within 5 minutes after being taken out, and particles with a cut-off particle size below 5µm were collected and then determined by a high performance liquid chromatography.

Unless otherwise specified, the vibrating screen atomizers used in the following examples and application example were as described in CN111569200A, the current was 1A, ± 2%; the voltage was 5V +/-2%.

Unless otherwise specified, the prescriptions of the tobramycin inhalation solutions used in the following examples were selected from the following two:
Prescription 1: tobramycin (6 w/v%), sodium chloride (0.225 w/v%), sulfuric acid (appropriate amount), sodium hydroxide (appropriate amount added if necessary), and water for injection. Sulfuric acid (appropriate amount) and sodium hydroxide (appropriate amount added if necessary) were used to adjust the pH value to 4.5-7.5;
Prescription 2: tobramycin (6 w/v%), sodium chloride (0.225 w/v%), citric acid (0.4 w/v%), sulfuric acid (appropriate amount), sodium hydroxide (appropriate amount added if necessary), and water for injection. Sulfuric acid (appropriate amount), sodium hydroxide (appropriate amount added if necessary) were used to adjust the pH value to 4.5-7.5.

### Example 1

Vibrating screen atomizers with different number of holes but with the same other parameters were selected to test the atomizing effect when they were applied to the tobramycin inhalation solution (Prescription 1), and the results were shown in table 1.

**Table 1 Influence of different number of holes on the atomization effect**

| | | No. 1 | No. 2 | No. 3 | No. 4 | No. 5 | No. 6 |
|---|---|---|---|---|---|---|---|
| Diameter of micropores /µm | | 4 | 4 | 4 | 4 | 4 | 4 |
| | Vibrating frequency KHz | 160 | 160 | 160 | 160 | 160 | 160 |
| | Pore size distribution (proportion of number of micropores with a micropore diameter of 3.8µm ∼ 4.2 µm based on total micropore numbers)/% | 85 | 85 | 85 | 85 | 85 | 85 |
| Taper/° | | 50 | 50 | 50 | 50 | 50 | 50 |
| | Number of micropores | 1200 | 1400 | 1500 | 1600 | 1800 | 2000 |
| | Atomization rate /ml/min | 0.62 | 0.55 | 0.52 | 0.51 | 0.47 | 0.35 |
| D₅₀/µm | | 4.6 | 3.5 | 3.3 | 3.0 | 2.5 | 1.9 |
| | Fine particle dose (FPD) (with an aerodynamic particle size of less than 5.39µm)/mg | 107.4 | 180.2 | 184.1 | 189.4 | 196.8 | 112.6 |
| FPF | | 39% | 63% | 74% | 79% | 81 % | 41 % |

### Example 2

Vibrating screen atomizers with different diameters of micropores but with the same other parameters were selected to test the atomizing effect when they were applied to the tobramycin inhalation solution (Prescription 1), and the results were shown in table 2.

**Table 2 Influence of different diameters of micropore on the atomization effect**

| | | No. 1 | No. 2 | No. 3 | No. 4 | No. 5 | No. 6 |
|---|---|---|---|---|---|---|---|
| Number of micropores | | 1600 | 1600 | 1600 | 1600 | 1600 | 1600 |
| Vibrating frequency KHz | | 160 | 160 | 160 | 160 | 160 | 160 |
| Pore size distribution (proportion of number of micropores with a micropore diameter of 3.8µm ~ 4.2 µm based on total micropore numbers)/% | | 85 | 85 | 85 | 85 | 85 | 85 |
| | Taper/° | 50 | 50 | 50 | 50 | 50 | 50 |
| | Diameters of micropores /µm | 3.3 | 3.8 | 3.9 | 4.0 | 4.2 | 4.7 |
| | Atomization rate /ml/min | 0.32 | 0.48 | 0.49 | 0.50 | 0.51 | 0.62 |
| D₅₀/µm | | 2.9 | 3.1 | 3.1 | 3.0 | 3.0 | 4.2 |
| FPD/mg | | 109.4 | 188.2 | 187.1 | 189.4 | 189.8 | 107.6 |
| FPF | | 40% | 77% | 75% | 77% | 78% | 41% |

### Example 3

Vibrating screen atomizers with different vibrating frequency but with the same other parameters were selected to test the atomizing effect when they were applied to the tobramycin inhalation solution (Prescription 2), and the results were shown in table 3.

**Table 3 Influence of different vibrating frequency on the atomization effect**

| | | No. 1 | No. 2 | No. 3 | No. 4 | No. 5 |
|---|---|---|---|---|---|---|
| | Vibrating frequency KHz | 90 | 110 | 130 | 160 | 180 |
| | Diameter of micropores/µm | 4 | 4 | 4 | 4 | 4 |
| | Number of micropores | 1600 | 1600 | 1600 | 1600 | 1600 |
| | Pore size distribution (proportion of number of micropores with a micropore diameter of 3.8µm ∼ 4.2 µm based on total micropore numbers)/% | 85 | 85 | 85 | 85 | 85 |
| Taper/° | | 50 | 50 | 50 | 50 | 50 |
| | Atomization rate /ml/min | 0.27 | 0.47 | 0.49 | 0.51 | 0.55 |
| D₅₀/µm | | 5.8 | 3.8 | 3.5 | 3.0 | 2.5 |
| FPD/mg | | 74.5 | 173.9 | 179.7 | 189.4 | 105.1 |
| FPF | | 27 % | 70% | 74 % | 77% | 40% |

### Example 4

Vibrating screen atomizers with different proportion of number of micropores with a micropore diameter of 3.8 ~ 4.2 µm based on total micropore numbers (pore size distribution) but with the same other parameters were selected to test the atomizing effect when they were applied to the tobramycin inhalation solution (Prescription 2), and the results were shown in table 4.

**Table 4 Influence of different pore size distribution on the atomization effect**

| | | No. 1 | No. 2 | No. 3 | No. 4 | No. 5 |
|---|---|---|---|---|---|---|
| | Pore size distribution (proportion of number of micropores with a micropore diameter of 3.8µm ∼ 4.2 µm based on total micropore numbers)/% | 50 | 70 | 80 | 85 | 100 |
| | Vibrating frequency KHz | 160 | 160 | 160 | 160 | 160 |
| | Number of micropores | 1600 | 1600 | 1600 | 1600 | 1600 |
| | Diameter of micropores / µm | 4 | 4 | 4 | 4 | 4 |
| Taper /° | | 50 | 50 | 50 | 50 | 50 |
| | Atomization Rate/ml/min | 0.48 | 0.47 | 0.52 | 0.51 | 0.51 |
| D₅₀/µm | | 4.6 | 4.2 | 3.5 | 3.2 | 3.0 |
| FPD/mg | | 73.5 | 103.9 | 179.7 | 189.4 | 197.1 |
| FPF | | 30% | 39% | 74% | 78% | 84% |

### Example 5

The effect of atomizing the tobramycin inhalation solution (Prescription 1) between a vibrating screen atomizer and a compressed air atomizer was compared, wherein the vibrating screen atomizer was an atomizer meeting the requirements of the present invention, the compressed air atomizer was an imported atomizer (Pari TurboBoy N), the two atomizers atomized liquid medicine in the same time, and the administration effect was compared, and the result was shown in Table 5.

**Table 5 Atomization effect of the vibrating screen atomizer and the compressed air atomizer**

| | Parameter(s) | D₅₀/µm | FPD/mg | FPF |
|---|---|---|---|---|
| Vibrating screen atomizer | Diameter of micropores: 4 µm | 3.2 | 189.2 | 78% |
| | Vibrating frequency: 160KHz | | | |
| | Number of micropores: 1600 | | | |
| | Pore size distribution (proportion of number of micropores with a micropore diameter of 3.8µm ∼ 4.2 µm based on total micropore numbers)/%:85% | | | |
| | Taper: 50 | | | |
| | Atomization rate: 0.51 ml/min | | | |
| Compressed air atomizer | Pump rate: 8L/min | 3.3 | 120.2 | 43 % |

### Example 6

Different brands of vibrating screen atomizers purchased in the market and the vibrating screen atomizers according to the present invention were selected to test the atomizing effect when they were applied to the tobramycin inhalation solution (Prescription 1), the parameters of the vibrating screen atomizers according to the present invention were shown in table 6, and the results were shown in table 7.

**Table 6 Parameters of the vibrating screen atomizers according to the present invention**

| | Parameter(s) |
|---|---|
| Vibrating screen atomizer | Diameter of micropores: 4 µm |
| | Vibrating frequency: 160KHz |
| | Number of micropores : 1600 |
| | Pore size distribution (proportion of number of micropores with a micropore diameter of 3.8µm ∼ 4.2 µm based on total micropore numbers)/%: 85% |
| | Taper: 50 |
| | Atomization rate: 0.51 ml/min |

**Table 7 Atomization effect of different vibrating screen atomizers**

| | Vibrating screen atomizers according to the present invention | YUWELL NM211C | OMRON NE-U22 | PHILIPS DK012 |
|---|---|---|---|---|
| D₅₀/µm | 3.2 | 4.2 | 3.6 | 4.7 |
| FPD/mg | 189.7 | 112.6 | 98.7 | 89.3 |
| FPF | 77% | 42% | 40% | 37% |

### Example 7

The parameters of the vibrating screen atomizers according to the present invention were shown in table 8, the tobramycin inhalation solution (Prescription 1) was used to test, the influence of different tapers on the atomization rate was examined, and the results were shown in table 9.

**Table 8 Parameters of the vibrating screen atomizers according to the present invention**

| | Parameter(s) |
|---|---|
| Vibrating screen atomizer | Diameter of micropores: 4 µm |
| | Vibration frequency: 160KHz |
| | Number of micropores: 1600 |
| | Pore size distribution (proportion of number of micropores with a micropore diameter of 3.8µm ∼ 4.2 µm based on total micropore numbers)/%: 85% |

**Table 9 Effect of different tapers on atomization rate**

| | No. 1 | No. 2 | No. 3 | No. 4 | No. 5 |
|---|---|---|---|---|---|
| Taper | 20 | 30 | 40 | 50 | 60 |
| Atomization Rate/ml/min | 0.15 | 0.47 | 0.48 | 0.51 | 0.46 |

### Examples 8 to 11

Prescription: tobramycin (6 w/v%), sodium chloride (0.225 w/v%), sulfuric acid (appropriate amount), sodium hydroxide (appropriate amount added if necessary), and water for injection, pH 6.2.

Process: 75kg of water for injection was added into a liquid preparation tank, the temperature was controlled not more than 60 °C, raw and auxiliary materials were added, and stirred to completely be dissolved, wherein the pH value was about 6.2. The formed solution was supplemented with water for injection to 100kg, stirred for 10min, and encapsulated in a 5ml low density polyethylene bottle. The droplet size was controlled, the delivery rate, total delivery amount and fine particle dose were determined. Table 10 showed the droplet size of examples 8-11.

**Table 10 droplet size of examples 8-11**

| | D₁₀/µm | D₅₀/µm | D₉₀/µm |
|---|---|---|---|
| Example 8 | 0.88 | 2.06 | 7.43 |
| Example 9 | 1.04 | 3.41 | 7.51 |
| Example 10 | 1.31 | 4.16 | 8.18 |
| Example 11 | 1.78 | 5.40 | 10.17 |

Delivery rate, total delivery amount: which were determined according to the provisions of [delivery rate and total delivery amount] under terms of 0111 inhalation liquid preparation in an inhalation solution, four general principles, Chinese Pharmacopoeia, 2020 edition.

Fine particle dose: which were measured according to 0951 determination method of aerodynamics properties of fine particles in inhalation solutions, four general principles, Chinese Pharmacopoeia, 2020 edition by using a NGI apparatus. The measurement results were shown in Table 11.

**Table 11 Delivery rate, total delivery amount, fine particle dose, and fine particle fraction of examples 8-11**

| Sample name | Delivery Rate (mg/min) | Total delivery amount (mg) | Fine particle dose (mg) | Fine particle fraction (%) |
|---|---|---|---|---|
| Example 8 | 6.4 | 105.7 | 171.4 | 69% |
| Example 9 | 5.9 | 104.8 | 191.4 | 79% |
| Example 10 | 6.8 | 102.6 | 163.8 | 67 % |
| Example 11 | 5.6 | 82.1 | 111.2 | 38 % |

| | | | | |
|---|---|---|---|---|
| Note: fine particle fraction = fine particle dose/delivery dose, wherein fine particle dose (FPD) refers to the dose of fine particles with an aerodynamic particle size of less than 5.39 microns. | | | | |

### Application example 1: clinical trial of tobramycin inhalation solution

### Test drugs:

Test groups: prescription of tobramycin inhalation solution: tobramycin (6 w/v%), sodium chloride (0.225 w/v%), sulfuric acid (appropriate amount), sodium hydroxide (appropriate amount added if necessary), and water for injection, pH 6.0.

### Control group: placebo, saline (0.9 w/v%)

### Dosage:

The product was for inhalation only. The recommended dosage was one (300 mg) each time, twice daily for 28 days, without adjusting the dosage according to body weight.

The application period of the product was 28 days, and the interval between two application periods should be 28 days, i.e. after the patients continuously taken the drugs for 28 days, stopped for 28 days, and then resumed.

The interval between two application periods daily should be as close as possible to 12 hours, and should be not less than 6 hours. If the drug was missed once, it should be replenished as soon as possible at least 6 hours before the next scheduled administration time. If the time was less than 6 hours from the next scheduled administration time, the drug was administered according to the originally scheduled administration time without need of supplement.

### Instructions for use:

A vibrating screen atomizer (whose parameters were shown in table 12) was used, and one bottle of the product was taken for each atomization. After unscrewing the cap of the bottle, the liquid medicine was completely extruded into an atomizing cup of the atomizer, the atomizer was turned on, and was inhaled through mouth until the atomization was complete (i.e., no fogging occured, about 10-15 minutes).

It is recommended that patients receiving various inhalation therapies inhale other drugs before inhalation of the product. The product can not be diluted or mixed with other drugs in the atomizer, and can not be used for subcutaneous, intravenous or intrathecal injection.

Before using the product, please carefully read the user's information manual to learn more about how to use the product, and follow the instructions on the use and maintenance of the vibrating screen atomizer. When inhaling the product, the patients should sit upright or stand upright, keep the atomizer upright and hold, and breathe normally through the mouthpiece/mask of the atomizer.

**Table 12 Parameters of the vibrating screen atomizer according to the present invention**

| | Parameter(s) |
|---|---|
| Vibrating screen atomizer | Diameter of micropores: 4 µm |
| | Vibration frequency: 160KHz |
| | Number of micropores: 1600 |
| | Pore size distribution (proportion of number of micropores with a micropore diameter of 3.8µm ∼ 4.2 µm based on total micropore numbers)/%: 85% |
| | Taper: 50 |
| | Atomization rate: 0.51 ml/min |

The project team conducted a multicenter, randomized, double-blind, basic therapy, placebo-controlled, parallel clinical study, to test the effectiveness and safety of tobramycin inhalation solution in the treatment for adult bronchodilation with pulmonary Pseudomonas aeruginosa infection. A total of 594 patients with bronchiectasis were screened in this study, and 357 patients were finally enrolled. The subjects who have completed all the examinations in the screening period and passed the evaluation by the researchers were interviewed for 2A sputum culture test. The subjects whose test results meet the inclusion standard were interviewed for 2B test. The subjects were randomly distributed to a test group or a control group to enter a treatment period. During the treatment period, they were administrated with the tobramycin inhalation solution (300mg/5ml/time and 2 times/day) or placebo (physiological saline) (5 ml/time and 2 times/day) respectively by inhalation, and both the test drugs and the control drugs were administrated for 28 days and stopped for 28 days in an intermittent administration mode, and were circulated for 2 times.

Two main efficacy endpoint indexes were used in the study: 1) change in load from baseline in sputum culture of Pseudomonas Aeruginosa (PA) on the 29th day after treatment; 2) change in QOL-B-RSS score from baseline on the 29th day after treatment.

Test results: for a modified intention-to-treat analysis (MITTS), covariance analysis was carried out by taking a baseline as a covariate, after correcting grouping factors, central factors, randomization control and missing value filling, the least square mean value of PA load in the test group (namely the technical solution according to the present invention) reduced by 2.50 Log10cfu/g (SE: 0.28), the least square mean value of PA load in the control group reduced by 0.76 Log10cfu/g (SE: 0.27), the difference between the two groups was 1.74 Log10cfu/g, 95% Cl was 1.12-2.35 (P < 0.001), and the central effect was not significant, this shows that the reduction value of PA load in the test group is more effective than that in the control group.

For MITTS, covariance analysis was carried out by taking a baseline as a covariate, after correcting grouping factors, central factors, randomization control and missing value filling, the least square mean value of QOL-B-RSS score in the test group (namely the technical solution according to the present invention) increased by 8.48 (SE: 0.97), the least square mean value of QOL-B-RSS score in the control group increased by 0.56 (SE: 0.97), the difference between the two groups was 7.91, 95% Cl was 5.72-10.11( P < 0.001 ), and the central effect was not significant, this shows that the QOL-B-RSS score in the test group is more effective than that in the control group.

The inventor carried out the above experiment aiming at prescription 2, and achieved the same clinical treatment effect as prescription 1.

## Claims

1. Use of a pharmaceutical assembly comprising a tobramycin inhalation solution in the manufacture of a pharmaceutical product for the treatment of bronchiectasis,
wherein the pharmaceutical assembly comprises:
(a) the tobramycin inhalation solution; and
(b) a vibrating screen atomizer matched with the tobramycin inhalation solution;
wherein the central area of metal mesh of the vibrating screen atomizer has 1400-1800 micropores;
the droplet sizeof the tobramycin inhalation solution is as follows: D₁₀ is 0.5 µm-2.5 µm, D₅₀ is 2.0 µm-4.2 µm, and D₉₀ is 6.0 µm-9.0 µm; the mass percentage of particles with an aerodynamic particle size of less than 5.39 µm in the tobramycin inhalation solution is not less than 45%.

2. The use according to claim 1, **characterized in that** the central area of metal mesh of the vibrating screen atomizer has 1600-1800 micropores.

3. The use according to claim 1, **characterized in that** the cross-section of the micropores in the vibrating screen atomizer is distributed in a stepped manner, and has a taper of 30∼50 degrees.

4. The use according to claim 1, **characterized in that** the number of the micropores having a diameter of 3.8µm ∼4.2 µm is not less than 80% based on the total number of the micropores.

5. The use according to claim 1, **characterized in that** the vibrating screen atomizer has a vibrating frequency of 110 KHz ∼160 KHz.

6. The use according to claim 1, **characterized in that** the vibrating screen atomizer has an atomization rate of 0.47 ml/min ∼0.55 ml/min.

7. The use according to claim 1, **characterized in that** the vibrating screen atomizer has a current of 1A, ± 2%; and a voltage of 5V, ± 2%.

8. The use according to claim 1, **characterized in that** the mass percentage of particles with an aerodynamic particle size of less than 5.39 µm in the tobramycin inhalation solution is 45%∼84%.

9. The use according to claim 1, **characterized in that** the droplet size of the tobramycin inhalation solution is as follows: D₁₀ is 0.5 µm-1.5 µm, D₅₀ is 3.0 µm-3.5 µm, and D₉₀ is 6.0 µm-8.5 µm.

10. The use according to claim 1, **characterized in that** the tobramycin inhalation solution contains 4 %∼ 8% (w/v) of tobramycin, 0.1 %∼ 0.9% (w/v) of sodium chloride, and has a pH of 4.5 - 7.5.

11. A pharmaceutical assembly comprising a tobramycin inhalation solution, the pharmaceutical assembly comprises:
(a) the tobramycin inhalation solution; and
(b) a vibrating screen atomizer matched with the tobramycin inhalation solution;
wherein the central area of metal mesh of the vibrating screen atomizer has 1400-1800 micropores;
the droplet size of the tobramycin inhalation solution is as follows: D₁₀ is 0.5 µm-2.5 µm, D₅₀ is2.0 µm-4.2 µm, and D₉₀ is 6.0 µm-9.0 µm; the mass percentage of particles with an aerodynamic particle size of less than 5.39 µm in the tobramycin inhalation solution is not less than 45%.

12. The pharmaceutical assembly according to claim 11, **characterized in that** the central area of metal mesh of the vibrating screen atomizer has 1600-1800 micropores.

13. The pharmaceutical assembly according to claim 11, **characterized in that** the cross-section of the micropores in the vibrating screen atomizer is distributed in a stepped manner, and has a taper of 30∼50 degrees.

14. The pharmaceutical assembly according to claim 11, **characterized in that** the number of the micropores having a diameter of 3.8∼4.2 µm is not less than 80% based on the total number of the micropores.

15. The pharmaceutical assembly according to claim 11, **characterized in that** the vibrating screen atomizer has a vibrating frequency of 110 KHz ∼160 KHz.

16. The pharmaceutical assembly according to claim 11, **characterized in that** the vibrating screen atomizer has an atomization rate of 0.47 ml/min ∼0.55 ml/min.

17. The pharmaceutical assembly according to claim 11, **characterized in that** the vibrating screen atomizer has a current of 1A, ± 2%; and a voltage of 5V, ± 2%.

18. The pharmaceutical assembly according to claim 11, **characterized in that** the mass percentage of the particles with an aerodynamic particle size of less than 5.39 mu in the tobramycin inhalation solution is 45%∼84%.

19. The pharmaceutical assembly according to claim 11, **characterized in that** the droplet size of the tobramycin inhalation solution is as follows: D₁₀ is 0.5-1.5 µm, D₅₀ is3.0-3.5 µm, and D₉₀ is 6.0-8.5 µm.

20. The pharmaceutical assembly according to claim 11, **characterized in that** the tobramycin inhalation solution contains 4% ∼ 8% (w/v) of tobramycin, 0.1% ∼ 0.9% (w/v) of sodium chloride, and has a pH of 4.5 - 7.5.
